(19) Europäisches Patentamt
European Patent Office
Office européen des brevets

(11) EP 2 777 688 A1

(12) EUROPEAN PATENT APPLICATION

(43) Date of publication:
**17.09.2014 Bulletin 2014/38**

(21) Application number: **13162464.5**

(22) Date of filing: **05.04.2013**

(51) Int Cl.:
*A61K 8/25* (2006.01) *A61K 8/26* (2006.01)
*A61Q 5/10* (2006.01) *A61K 8/73* (2006.01)
*A61K 8/86* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **11.03.2013 US 201361775925 P**

(71) Applicant: **The Procter & Gamble Company**
**Cincinnati, OH 45202 (US)**

(72) Inventors:
- **Hamersky, Mark, William**
**Cincinnati, OH Ohio 45217 (US)**
- **Klingelmeyer, Annette**
**64295 Darmstadt (DE)**
- **Krause, Thomas**
**64295 Darmstadt (DE)**
- **Niesig, Silke**
**64295 Darmstadt (DE)**
- **Sunkel, Jorge Max**
**Mason, OH Ohio 45040-9462 (US)**

(74) Representative: **Holmes, Rosalind**
**Procter & Gamble Service GmbH**
**Legal Innovation**
**Patent Department**
**Frankfurter Straße 145**
**61476 Kronberg im Taunus (DE)**

# (54) Method for colouring hair

(57) A method for colouring hair. The method comprises the formation of a first plurality of coated hair fibre portions by applying a first composition to a first plurality of hair fibre portions and subsequently styling the hair. The first plurality of coated hair fibre portions is contacted with a second plurality of coated hair fibre portions but does not comprise the application of a solid barrier means in order to separate the first plurality of coated hair fibre portions from the second plurality of coated hair fibre portions. The composition comprises from about 0.2% to about 20% of viscosity-increasing agent and the viscosity-increasing agent is selected from the group consisting of: clays; polysaccharides; silicas; block copolymers of polypropylene oxide (PPO) and polyethylene oxide (PEO); and mixtures thereof. Also provided are a hair colouring composition being in ready to use form, the use of the composition, a developing formulation, and a process for creating a hair colouring composition.

**FIG. 1**

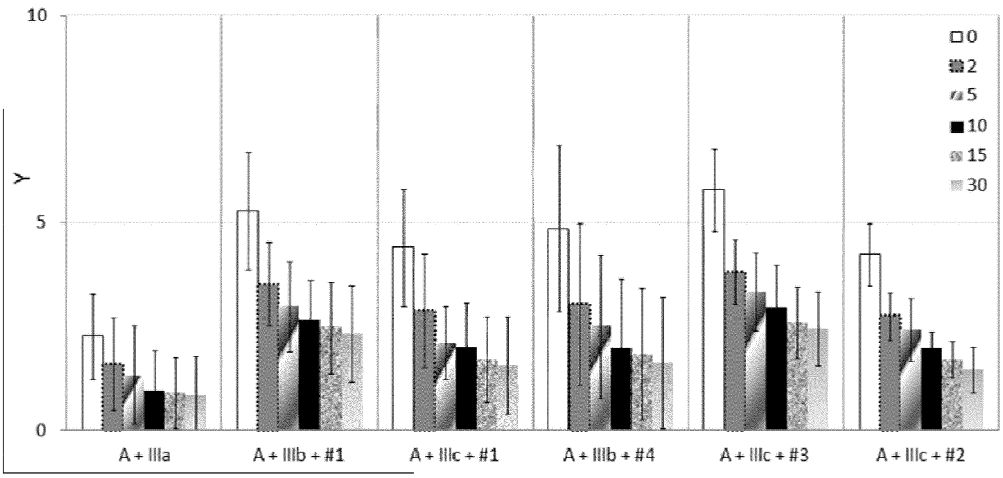


EP 2 777 688 A1

## Description

FIELD OF THE INVENTION

A method for colouring hair wherein the method comprises the formation of a first plurality of coated hair fibre portions and subsequently styling the hair wherein the first plurality of coated hair fibre portions is contacted with a second plurality of coated hair fibre portions wherein the method does not comprise the application of a solid barrier means in order to separate the first plurality of coated hair fibre portions from the second plurality of coated hair fibre portions.

BACKGROUND OF THE INVENTION

Hair colouring or dyeing involves the application of a hair dye onto hair which results in the colouration of hair fibres. Typically the hair colour is changed or 'freshened up'. In highlighting, a limited number of sections of the head of hair - typically a plurality of hair fibres from their route to tip - are dyed to a lighter hair colour, wherein the sections are spaced out at intervals such that un-dyed sections remain in between. Lowlighting is a similar procedure wherein a darker colour dye is utilised instead. The hair can also be highlighted with other colours e.g. red and/or purple tones. The entire head of hair can be dyed using this method e.g. with 3 different hair colouring agents for a more striking look. The end result is normally increased appearance of texture and vibrancy of the hair. The dyeing can also be tailored to the final hairstyle in order to highlight certain aspects or draw attention away from other features. Subtle highlighting/lowlighting can give the impression of a slight lightening/darkening of the hair shade and results in a fresher look.

Highlighting (and lowlighting) typically employs the use of barrier means, such as foils, in order to prevent bundles of hair fibres intentionally treated with a hair colouring agent from contacting other hair fibres and thus transfer of the hair colouring agent onto hair fibres that were not intended to be coloured at all or intended to be coloured with a different dye. Coloured fibres when contacted with other hair fibres can transfer their dye onto these other fibres, which are then also dyed - this is sometimes known as 'staining'. Therefore, barrier means are used to wrap up each intentionally dyed bundle of hair fibres and thus separate it from the other hair fibres. The wrapped bundles are then typically left to develop for a period of time before the hair dye is rinsed out and the final cut and style carried out.

There is a need, however, for the consumer to feel more beautiful during the hair dyeing process - some consumers believe that it detracts from this when they have to spend a period of time in the hairdressing salon with their head covered in e.g. foil parcels. Moreover, there is a need for speeding up the process of dyeing hair. Furthermore, there is a need for providing the stylist with greater artistic and creative freedom, vis-à-vis the relationship of the hair colour and the final hairstyle, during the application of the dye and during the dye development time. There is also a need for reducing the use of solid barrier means e.g. foils, such as for environmental reasons e.g. reduction of waste. Furthermore, there is a need to provide the consumer with a means to highlight/lowlight their hair by themselves i.e. at home without the need for a stylist.

In summary, there is a constant need for providing methods resulting in improved efficiency, flexibility and freedom for the stylist. There is a need for the consumer to have an improved feeling of well-being and beautification during the entire process of hair colouring and not just after treatment, and for the process to be quicker. There is a need for the stylists to be able to envision, create and experiment with the final look at every stage throughout the colouring process.

SUMMARY OF THE INVENTION

According to the first aspect, the invention relates to a method for colouring hair, wherein the method comprises:

(i) the formation of a first plurality of coated hair fibre portions, by applying a first composition to a first plurality of hair fibre portions;
wherein the first composition comprises a first hair colouring agent;
and subsequently
(ii) styling the hair wherein the first plurality of coated hair fibre portions is contacted with a second plurality of coated hair fibre portions;
wherein the second plurality of coated hair fibre portions are coated with a second composition;

wherein the second composition comprises a second hair colouring agent; wherein the method does not comprise the application of a solid barrier means in order to separate the first plurality of coated hair fibre portions from the second plurality of coated hair fibre portions;
wherein the first composition and the second composition are substantially free of persulfate;

and wherein the first composition and the second composition comprise from about 0.2% to about 20% of viscosity-increasing agent, by total weight of the respective composition;
and wherein the viscosity-increasing agent is selected from the group consisting of: clays; polysaccharides; silicas; block copolymers of polypropylene oxide (PPO) and polyethylene oxide (PEO); and mixtures thereof.

According to a second aspect, the present invention relates to a hair colouring composition being in ready to use form; wherein the composition comprises a hair colouring agent, an oxidising agent, a viscosity-increasing agent, water, and optionally a fatty alcohol; wherein the hair colouring agent is a mixture of at least one primary intermediate and at least one coupler; and wherein the viscosity-increasing agent is selected from the group consisting of: clays; polysaccharides; silicas; block copolymers of polypropylene oxide (PPO) and polyethylene oxide (PEO); and mixtures thereof; and wherein the composition comprises from about 0.2% to about 20% of viscosity-increasing agent.

According to a third aspect, the present invention relates to the use of the composition according to the second aspect, for colouring and/or styling hair.

According to a fourth aspect, the present invention relates to a developing formulation comprising:

- from about 0.5% to about 10% viscosity-increasing agent and wherein the viscosity-increasing agent is selected from the group consisting of: clays; polysaccharides; silicas; block copolymers of polypropylene oxide (PPO) and polyethylene oxide (PEO); and mixtures thereof;
- from about 1% to about 15% of an oxidising agent;
- optionally from about 1% to about 10% of a fatty alcohol;
- optionally from about 0.1% to about 2% of a surfactant;
- water.

According to a fifth aspect, the present invention relates to a process for creating a hair colouring composition comprising mixing:

a) a developing formulation according to the fourth aspect;
b) a hair colour formulation comprising a hair colouring agent wherein the hair colouring agent is a mixture of at least one primary intermediate and at least one coupler.

According to a sixth aspect, the invention relates to a kit comprising: (a) application instructions comprising the method according to the first aspect; (b) a composition.

BRIEF DESCRIPTION OF THE DRAWINGS

Figs 1 and 2: Show percentage hold data from experiment 1. The axis labelled Y indicates percentage hold. Different compositions are compared differing in terms of the viscosity-increasing agent present and/or its amount. For each composition, percentage hold after a certain number of minutes was calculated (from 0 to 30 min). Fig. 1 and Fig. 2 differ from each other by the colour formulation used. See the Examples and Data sections for further detail.

DETAILED DESCRIPTION OF THE INVENTION

In all embodiments of all aspects of the present invention, all percentages are by weight of the total composition or formulation, unless specifically stated otherwise. All ratios are weight ratios, unless specifically stated otherwise. References to 'parts' e.g. a mixture of 1 part X and 3 parts Y, is by weight unless stated otherwise. "QS" or "QSP" means sufficient quantity for 100%. +/- indicates the standard deviation. All ranges are inclusive and combinable. The number of significant digits conveys neither a limitation on the indicated amounts nor on the accuracy of the measurements. All numerical amounts are understood to be modified by the word "about" unless otherwise specifically indicated. Unless otherwise indicated, all measurements are understood to be made at 23°C and at ambient conditions, where "ambient conditions" means conditions at about one atmosphere (atm) of pressure and at about 50% relative humidity. Herein "min" means "minute" or "minutes". Herein "mol" means mole. Herein "g" following a number means "gram" or "grams" unless stated otherwise. All weights as they pertain to listed ingredients are based on the active level and do not include carriers or by-products that may be included in commercially available materials, unless otherwise specified. Herein, "comprising" means that other steps not listed and other ingredients not listed can be added. "Comprising" encompasses the terms "consisting of'' and "consisting essentially of''. The compositions, formulations, methods, uses, kits, and processes of the present invention can comprise, consist of, and consist essentially of the elements and limitations of the invention described herein, as well as any of the additional or optional ingredients, components, steps, or limitations described herein. The term "molecular weight" or "M.Wt." or "MW" and the like as used herein means the number average molecular weight unless otherwise stated. All percentages are calculated by weight unless otherwise stated. Embodiments and aspects described herein may comprise or be combinable with elements, features or components of other embodiments and/or aspects despite not being expressly exemplified in combination, unless otherwise stated or an incompatibility is stated.

The term "substantially free from" or "substantially free of'' as used herein means less than about 1%, or less than 0.8%, or less than 0.5%, or less than 0.3%, or about 0%, by total weight of the composition or formulation.

"Hair", as used herein, means mammalian hair including scalp hair, facial hair and body hair, more preferably hair on the human head and scalp. "Hair shaft" or "hair fibre" means an individual hair strand and may be used interchangeably with the term "hair." "Proximal to the scalp," as used herein, means that portion of an extended, or substantially straightened, hair shaft that is closer in distance to the scalp than to the end of the hair. Thus, about 50% of the hair would be considered proximal to the scalp, and about 50% of the hair would be distal to the scalp. "z cm proximal to the scalp" means a distance "z" along the hair, with one endpoint being on or directly adjacent to the scalp, and the second endpoint being measured "z" centimetres along the length of the extended or substantially straightened hair.

"Cosmetically acceptable," as used herein, means that the compositions, formulations or components described are suitable for use in contact with human keratinous tissue without undue toxicity, incompatibility, instability, allergic response, and the like. All compositions and formulations described herein which have the purpose of being directly applied to keratinous tissue are limited to those being cosmetically acceptable.

"Derivatives," as used herein, includes but is not limited to, amide, ether, ester, amino, carboxyl, acetyl, acid and/or alcohol derivatives of a given compound.

"Monomer," as used herein, means a discrete, non-polymerised chemical moiety capable of undergoing polymerisation in the presence of an initiator or any suitable reaction that creates a macromolecule e.g. such as polycondensation, polyaddition, anionic or cationic polymerization. "Unit", as used herein, means a monomer that has already been polymerised i.e. is part of the polymer.

"Polymer," as used herein, means a chemical formed from the polymerisation of two or more monomers. The term "polymer" as used herein shall include all materials made by the polymerisation of monomers as well as natural polymers. Polymers made from only one type of monomer are called homopolymers. A polymer comprises at least two monomers. Polymers made from two or more different types of monomers are called copolymers. The distribution of the different monomers can be random, alternating or block-wise (i.e. block copolymer). Except if stated otherwise, the term "polymer" used herein includes any type of polymer including homopolymers and copolymers.

"Associative thickening polymers" are polymers that are based on water-soluble polymers. These can be acrylate polymers, cellulose ethers or, polyethylene glycol. These typically comprise sidechains that are capped with water-insoluble hydrophobic groups like fatty alcohols, for example. In an aqueous solution or in emulsion, these polymers form a network that increases the viscosity of the solution/emulsion. The water-soluble backbone polymer is dissolves in water. The hydrophobic caps are adsorbed onto the hydrophobic emulsion polymer particles, or they form micelle structures with hydrophobes from other polymers. As each associative thickening polymer contains at least two hydrophobic caps, the result is a three-dimensional network within the emulsion. This increases the viscosity. Mainly the high- and mid-shear viscosity is affected.

"Kit" as used herein, means a package comprising a plurality of components. "Kit" may be referred to as "kit-of-parts". An example of a kit is, for example, a first composition and a separately packaged second composition and optionally application instructions.

"Implement", as used herein, means a device used to facilitate application of a composition to the hair and/or manipulation of the hair.

"Ready to use form" , as used herein, means no modification need be made e.g. no other composition/formulation need be added to it in order for it to provide its function. Thus a "hair colouring composition being in ready to use form'' means that no e.g. developing formulation need be mixed in or any other modification be made in order for the composition to provide its hair colouring effect on hair.

The inventors have answered the aforementioned needs by carefully selecting the specific combination of mutually compatible features such that the interaction therewith results in a method which provides the following benefits. Firstly, the method allows the stylist to design the colouring to the final hairstyle by creating the hairstyle <u>during</u> the application of the hair colouring agent. This ability is due to the sculptability and hold benefits afforded by the composition(s) as described herein. The stylist therefore has greater artistic freedom to envisage the final hairstyle and the hair colouring effects fitting optimally thereto. In addition, hold provided by the composition provides the stylist with security and self-confidence because the section of hair coloured can be put in a specific orientation or placed in a specific location and it remains in this orientation/location. The stylist is thus able to achieve a wide variety of complex hairstyles, which may or may not be related to the hairstyle result when the hair is dry and the salon visit is over. The method saves waste because the use of solid barrier means e.g. aluminium foils, is not necessary, since the hold/sculptability provided by the composition creates sufficient separation. The lack of foils also means that the stylist can better see where he or she is applying the colouring agent and where this is in relation to the entire head of hair. The method is also faster to execute than with foils. The method also provides an aesthetic improvement of the application process - a variety of hair

styles can be created during the application of the hair colouring agent. Furthermore, the method is easier for apprentice/trainee stylists to learn - highlighting with foils requires excellent technique and significant practice - whereas the method according to the present invention is much faster to learn. Moreover, the method provides a way for consumers to create simple high- or low-lights at home since use of foils at home can be impractical and difficult to apply with one pair of hands.

Without being bound by theory, it is believed that when the selected viscosity-increasing agent(s) is mixed in the amounts detailed herein with the other components of the composition the visco-elastic properties of the final composition are improved, in other words results in a stiffer and more elastic composition. In particular, it is thought that the viscosity-increasing agent(s) interacts with the hydrophilic phase of the a lamellar structure of the composition. The stiffer and more elastic properties of the composition mean that the mobility of the composition is reduced - it is less able to flow and slide over itself, which provides hold and sculptability to hair fibres coated with the composition. This hold and sculptability provides the stylist with much greater creative freedom.

The method does not comprise the application of a solid barrier means in order to separate the first plurality of coated hair fibre portions from the second plurality of hair fibre portions. Without being bound by theory, it is believed that the altered stiffness and elastic properties reduces the miscibility of the hair colouring agent and also results in slower diffusion of the hair colouring agent. This means that a solid barrier means can be avoided since minimal or no staining occurs. "Staining" as used herein, means the unintentional dyeing of hair, typically due to unintended contact of the hair with a hair colouring agent. Staining may result from the transfer/migration of a colouring agent from one plurality of hair fibres to another plurality of hair fibres.

The features of the method according to the first aspect, as well as the other aspects and other relevant components, are described in detail hereinafter.

The method of the first aspect details a first composition and a second composition. The first composition and the second composition respectively comprise a hair colouring agent. The hair colouring agent may be selected from the group consisting of: direct dyes, oxidative dye compounds, and mixtures thereof. In an embodiment, the first and/or second composition is obtained by mixing together a colour formulation, a thickening formulation, and a developing formulation. In an embodiment, the first and/or second composition is obtained by mixing together a colour formulation and a developing formulation or a thickening formulation. In an embodiment, the first and/or second composition is obtained by mixing together a colour formulation and a developing formulation, wherein the colour formulation comprises a hair colouring agent, and wherein the developing formulation comprises an oxidising agent and the viscosity-increasing agent.

The first composition and/or the second composition may comprise a direct dye. In an embodiment, the hair colouring agent, for example the first hair colouring agent and/or the second hair colouring agent, is a direct dye. The direct dye may be present in an amount of from about 0.001% to about 4%, or from about 0.005% to about 3%, or from about 0.01% to about 2%, by total weight of the colour formulation or the first/second composition. The presence of a direct dye and the proportion thereof is useful in that it can provide or enhance colouring/dyeing, particularly with regard to intensity. The direct dye may be selected from the group consisting of: nitro dyes to provide a blue colour, nitro dyes to provide a red colour, nitro dyes to provide a yellow colour, quinone dyes, basic dyes, neutral azo dyes, acid dyes, and mixtures thereof. In an embodiment, the direct dye is a nitro dye to provide a blue colour. In an embodiment, the direct dye is a nitro dye to provide a red colour. In an embodiment, the direct dye is a nitro dye to provide a yellow colour. In an embodiment, the direct dye is a quinone dye. In an embodiment, the direct dye is a basic dye. In an embodiment, the direct dye is a neutral azo dye. In an embodiment, the direct dye is an acid dye. In an embodiment, the direct dye is selected from the group consisting of: Acid dyes such as Acid Yellow 1, Acid Orange 3, Acid Black 1, Acid Black 52, Acid Orange 7, Acid Red 33, Acid Yellow 23, Acid Blue 9, Acid Violet 43, Acid Blue 16, Acid Blue 62, Acid Blue 25, Acid Red 4, Basic Dyes such as Basic Brown 17, Basic Red 118, Basic Orange 69, Basic Red 76, Basic Brown 16, Basic Yellow 57, Basic Violet 14, Basic Blue 7, Basic Blue 26, Basic Red 2, Basic Blue 99, Basic Yellow 29, Basic Red 51, Basic Orange 31, Basic Yellow 87, 4-(3-(4-amino-9,10-dioxo-9,10-dihydroanthracen-1-ylamino)propyl)-4-methylmorpholin-4-ium-methylsulfate, (*E*)-1-(2-(4-(4,5-dimethylthiazol-2-yl)diazenyl)phenyl)(ethyl)amino)ethyl)-3-methyl-1H-imidazo1-3-ium chloride, (*E*)-4-(2-(4-(dimethylamino)phenyl)diazenyl)-1-methyl-1H-imidazol-3-ium-3-yl)butane-1-sulfonate, (*E*)-4-(4-(2-methyl-2-phenylhydrazono)methyl)pyridinium-1-yl)butane-1-sulfonate, N,N-dimethyl-3-(4-(methylamino)-9,10-dioxo-4a,9,9a, 10-tetrahydroanthracen-1-ylamino)-N-propylpropan-1-aminium bromide, Disperse Dyes such as Disperse Red 17, Disperse Violet 1, Disperse Red 15, Disperse Violet 1, Disperse Black 9, Disperse Blue 3, Disperse Blue 23, Disperse Blue 377, Nitro Dyes such as 1-(2-(4-nitrophenylamino)ethyl)urea, 2-(4-methyl-2-nitrophenylamino)ethanol, 4-nitrobenzene-1,2-diamine, 2-nitrobenzene-1,4-diamine, Picramic acid, HC Red No. 13, 2,2'-(2-nitro-1,4-phenylene)bis(azanediyl)diethanol, HC Yellow No. 5, HC Red No. 7, HC Blue No.2, HC Yellow No. 4, HC Yellow No. 2, HC Orange No. 1, HC Red No. 1, 2-(4-amino-2-chloro-5-nitrophenylamino)ethanol, HC Red No. 3, 4-amino-3-nitrophenol, 4-(2-hydroxyethylamino)-3-nitrophenol, 2-amino-3-nitrophenol, 2-(3-(methylamino)-4-nitrophenoxy)ethanol, 3-(3-amino-4-nitrophenyl)propane-1,2-diol, HC Yellow No. 11, HC Violet No. 1, HC Orange No. 2, HC Orange No. 3, HC Yellow No. 9, HC Red No. 10, HC Red No. 11, 2-(2-hydroxyethylamino)-4,6-dinitrophenol, HC Blue No. 12, HC

Yellow No. 6, HC Yellow No. 12, HC Blue No. 10, HC Yellow No. 7, HC Yellow No. 10, HC Blue No. 9, 2-chloro-6-(ethylamino)-4-nitrophenol, 6-nitropyridine-2,5-diamine, HC Violet No. 2, 2-amino-6-chloro-4-nitrophenol, 4-(3-hydroxypropylamino)-3-nitrophenol, HC Yellow No. 13, 6-nitro-1,2,3,4-tetrahydroquinoxaline, HC Red No. 14, HC Yellow No. 15, HC Yellow No. 14, N2-methyl-6-nitropyridine-2,5-diamine, N1-allyl-2-nitrobenzene-1,4-diamine, HC Red No. 8, HC Green No.1, HC Blue No. 14, and Natural dyes such as Annato, Anthocyanin, Beetroot, Carotene, Capsanthin, Lycopene, Chlorophyll, Henna, Indigo, Cochineal.

The first composition and/or the second composition may comprise an oxidative dye compound. In an embodiment, the first and/or second composition comprise a hair colouring agent, wherein the hair colouring agent is an oxidative dye compound. In an embodiment, the first and/or second hair colouring agent are oxidative dye compounds; and wherein the first and/or second composition comprises an oxidising agent. The oxidative dye compound may be selected from the group consisting of: primary intermediates, couplers, and mixtures thereof. In an embodiment, the hair colouring agent is a mixture of at least one primary intermediate and at least one coupler. The oxidative dye compound may also be in the form of an oxidatively stable direct dye. In an embodiment, the hair colouring agent is a mixture of at least one primary intermediate, at least one coupler and at least one oxidative stable direct dye. The oxidative dye compounds suitable for use in composition(s)/formulation(s) described herein, in so far as they are bases, may be used as free bases or in the form of their physiologically compatible salts with organic or inorganic acids, such as hydrochloric, hydrobromic, citric, acetic, lactic, succinic, tartaric, or sulfuric acids, or, in so far as they have aromatic hydroxyl groups, in the form of their salts with bases, such as alkali phenolates.

Oxidative dye compounds are known in the art, and include aromatic diamines, aminophenols, aromatic diols and their derivatives (a representative but not exhaustive list of oxidation dye precursors can be found in Sagarin, "Cosmetic Science and Technology", "Interscience, Special Edn. Vol. 2 pages 308 to 310). It is to be understood that the primary intermediates and couplers (also collectively known as oxidative dye precursors) detailed below are only by way of example and are not intended to limit the compositions and processes herein. The primary intermediates and couplers may be used in the form of salts.

In an embodiment, the primary intermediate is selected from the group consisting of: toluene-2,5-diamine, p-phenylenediamine, *N*-phenyl-p-phenylenediamine, *N,N*-bis(2-hydroxyethyl)-p-phenylenediamine, 2-hydroxyethyl-p-phenylenediamine, hydroxypropyl-bis-(N-hydroxyethyl-p-phenylenediamine), 2-methoxymethyl-p-phenylenediamine, 2-(1,2-dihydroxyethyl)-p-phenylenediamine, 2,2'-(2-(4-aminophenylamino)ethylazanediyl)diethanol, 2-(2,5-diamino-4-methoxyphenyl)propane-1,3-diol, 2-(7-amino-2H-benzo[b][1,4]oxazin-4(3H)-yl)ethanol, 2-chloro-p-phenylenediamine, p-aminophenol, p-(methylamino)phenol, 4-amino-m-cresol, 6-amino-m-cresol, 5-ethyl-o-aminophenol, 2-methoxy-p-phenylenediamine, 2,2'-methylenebis-4-aminophenol, 2,4,5,6-tetraminopyrimidine, 2,5,6-triamino-4-pyrimidinol, 1-hydroxyethyl-4,5-diaminopyrazole sulfate, 4,5-diamino-1-methylpyrazole, 4,5-diamino-1-ethylpyrazole, 4,5-diamino-1-isopropylpyrazole, 4,5-diamino-1-butylpyrazole, 4,5-diamino-1-pentylpyrazole, 4,5-diamino-1-benzylpyrazole, 2,3-diamino-6,7-dihydropyrazolo[1,2-a]pyrazol-1(5H)-one dimethosulfonate, 4,5-diamino-1-hexylpyrazole, 4,5-diamino-1-heptylpyrazole, methoxymethyl-1,4-diaminobenzene, and mixtures thereof. In an embodiment, the primary intermediate is 2-methoxymethyl-1,4-benzenediamine. 2-methoxymethyl-1,4-benzenediamine is useful due to its improved (i.e. reduced) scalp sensitisation profile, which is relevant in view of the present method comprising a styling step.

In an embodiment, the coupler is selected from the group consisting of: 2,6-dihydroxyethylaminotoluene, resorcinol, 4-chlororesorcinol, 2-chlororesorcinol, 2-methylresorcinol, 4,6-dichlorobenzene-1,3-diol, 2,4-dimethylbenzene-1,3-diol, m-aminophenol, 4-amino-2-hydroxytoluene, 2-methyl-5-hydroxyethylaminophenol, 3-amino-2,6-dimethylphenol, 3-amino-2,4-dichlorophenol, 5-amino-6-chloro-o-cresol, 5-amino-4-chloro-o-cresol, 6-hydroxybenzomorpholine, 2-amino-5-ethylphenol, 2-amino-5-phenylphenol, 2-amino-5-methylphenol, 2-amino-6-methylphenol, 2-amino-5-ethoxyphenol, 5-methyl-2-(methylamino)phenol, 2,4-diaminophenoxyethanol, 2-amino-4-hydroxyethylaminoanisole, 1,3-bis-(2,4-diaminophenoxy)-propane, 2,2'-(2-methyl-1,3-phenylene)bis(azanediyl)diethanol, benzene-1,3-diamine, 2,2'-(4,6-diamino-1,3-phenylene)bis(oxy)diethanol, 3-(pyrrolidin-1-yl)aniline, 1-(3-(dimethylamino)phenyl)urea, 1-(3-aminophenyl)urea, 1-naphthol, 2-methyl-1-naphthol, 1,5-naphthalenediol, 2,7-naphthalenediol or 1-acetoxy-2-methylnaphthalene, 4-chloro-2-methylnaphthalen-1-ol, 4-methoxy-2-methylnaphthalen-1-ol, 2,6-dihydroxy-3,4-dimethylpyridine, 2,6-dimethoxy-3,5-pyridinediamine, 3-amino-2-methylamino-6-methoxypyridine, 2-amino-3-hydroxypyridine, 2,6-diaminopyridine, pyridine-2,6-diol, 5,6-dihydroxyindole, 6-hydroxyindole, 5,6-dihydroxyindoline, 3-methyl-1-phenyl-1*H*-pyrazol-5(4*H*)-one, 1,2,4-trihydroxybenzene, 2-(benzo[d][1,3]dioxol-5-ylamino)ethanol (also known as hydroxyethyl-3,4-methylenedioxyaniline), and mixtures thereof. In an embodiment, the coupler is 5-amino-4-chloro-o-cresol. In an embodiment, the coupler is 2,6-diaminopyridine. In an embodiment, the coupler is 2,6-dihydroxyethylaminotoluene.

In an embodiment, the hair colouring agent is a mixture of at least one primary intermediate and at least one coupler, wherein the primary intermediate is 2-methoxymethyl-1,4-diaminobenzene and wherein the coupler is selected from the group consisting of: 5-amino-4-chloro-o-cresol, 2,6-diaminopyridine, 2,6-dihydroxyethylaminotoluene, and mixtures thereof. In an embodiment, the hair colouring agent is a mixture of oxidative dye compounds, wherein the oxidative dye compounds are selected from the group consisting of: 6-hydroxyindole, 1,5-naphthalenediol, 2,7-naphthalenediol, hydroxybenzomorpholine, 2,4,5,6-tetraminopyrimidine, 1,3-bis-(2,4-diaminophenoxy)-propane, 2,6-dihydroxy-3,4-dimethylpyridine, dihydroxyindole, dihydroxyindoline, 2,3-diaminodihydropyrazolopyrazolone dimethosulfonate, 5-amino-6-chloro-o-cresol, and 3-amino-2,4-dichlorophenol.

The primary intermediates, couplers and mixtures thereof may be present in an amount of from about 0.001 % to about 12%, or from about 0.01 % to about 10%, or from about 0.05% to about 9%, or from about 1% to about 6%, by total weight of the composition or colour formulation. In an embodiment, the composition and/or formulation are substantially free of a direct dye.

In an embodiment, the first and/or second composition comprises an oxidizing agent. The oxidizing agent may be present in an amount sufficient to bleach melanin pigment in hair and/or cause formation of dye chromophores from oxidative dye compounds (including primary intermediates and/or couplers, when present). In an embodiment, the thickening formulation and/or the developing formulation comprise an oxidising agent. In an embodiment, the oxidising agent is present in an amount of from about 0.1% to about 20%, or from about 0.5% to about 12%, or from about 1% to about 10%, or from about 3% to about 10%, or from about 5% to about 10%. In an embodiment, the oxidising agent is present in an amount of from about 0.1 % to about 20%, or from about 1% to about 10%, or from about 2% to about 5%. Inorganic peroxygen materials capable of yielding hydrogen peroxide in an aqueous medium may be used. In an embodiment, the oxidising agent is selected from group consisting of: hydrogen peroxide; inorganic alkali metal peroxides (e.g. sodium periodate and sodium peroxide); organic peroxides (e.g. urea peroxide, melamine peroxide); inorganic perhydrate salt bleaching compounds (e.g. alkali metal salts of perborates, percarbonates, perphosphates, persilicates, and persulphates, particularly sodium salts thereof), which may be incorporated as monohydrates, tetrahydrates, etc.; alkali metal bromates; enzymes; and mixtures thereof. In an embodiment, the oxidizing agent is a percarbonate (such as sodium percarbonate, ammonium percarbonate and potassium percarbonate). In another embodiment, the oxidizing agent is sodium percarbonate. The first composition and second composition are substantially free of persulfate. In an embodiment, all compositions and formulations are substantially free of persulfate. In an embodiment, the method does not encompass or include bleaching the hair.

A composition and/or formulation as described herein may comprise at least one source of peroxymonocarbonate ions, e.g. *formed in situ* from a source of hydrogen peroxide and a carbonate ion source. The composition/formulation thus also may comprise a source of carbonate ions or carbamate ions or hydrocarbonate ions or any mixture thereof. The source may be selected from the group consisting of: sodium, potassium, guanidine, arginine, lithium, calcium, magnesium, barium, ammonium salts of carbonate, carbamate and hydrocarbonate ions, and mixtures thereof. Examples of mixtures thereof are: sodium carbonate, sodium hydrogen carbonate, potassium carbonate, potassium hydrogen carbonate, guanidine carbonate, guanidine hydrogen carbonate, lithium carbonate, calcium carbonate, magnesium carbonate, barium carbonate, ammonium carbonate, ammonium hydrogen carbonate, and mixtures thereof. Percarbonate salts may also be utilized to provide both the source of carbonate ions and oxidizing agent. The source of carbonate ions, carbamate and hydrocarbonate ions may be selected from the group consisting of: sodium hydrogen carbonate, potassium hydrogen carbonate, ammonium carbamate, and mixtures thereof.

A composition and/or formulation as described herein may comprise a radical scavenger, in a sufficient amount to reduce damage to the hair during an oxidative bleaching or colouring process. The radical scavenger is preferably selected such that it is not an identical species to an alkalising agent. The radical scavenger is a species that can react with a carbonate radical to convert the carbonate radical by a series of fast reactions to a less reactive species. The radical scavenger may be selected from the classes of: alkanolamines, amino sugars, amino acids, and mixtures thereof. The radical scavenger may be selected from the group consisting of: monoethanolamine, 3-amino-1-propanol, 4-amino-1-butanol,5-amino-1-pentanol, 1-amino-2-propanol, 1-amino-2-butanol, 1-amino-2-pentanol, 1-amino-3-pentanol, 1-amino-4-pentanol, 3-amino-2-methylpropan-1-ol, 1-amino-2-methylpropan-2-ol, 3-aminopropane-1,2-diol, glucosamine, N-acetylglucosamine, glycine, arginine, lysine, proline, glutamine, histidine, serine, tryptophan, and potassium, sodium and ammonium salts of the above, and mixtures thereof. In an embodiment, the radical scavenger compound is selected from the group consisting of: benzylamine, glutamic acid, imidazole, di-tert-butylhydroxytoluene, hydroquinone, catechol, and mixtures thereof.

A composition and/or formulation as described herein may comprise a chelant in an amount sufficient to reduce the amount of metals available to interact with formulation components, e.g. oxidizing agents, more particularly peroxides. Chelants are also known as chelators. The chelant for use herein may be selected from the group consisting of: diamine-N,N'-dipolyacid, monoamine monoamide-N,N'-dipolyacid, and N,N'-bis(2-hydroxybenzyl)ethylenediamine-N,N'-diacetic acid chelants (e.g. EDDS (ethylenediaminedisuccinic acid)), carboxylic acids (e.g. aminocarboxylic acids), phosphonic acids (e.g. aminophosphonic acids), polyphosphoric acids (in particular straight polyphosphoric acids), salts and derivatives thereof, and mixtures thereof. In an embodiment, the chelant is ethylenediamine tetraacetic acid (EDTA) and/or editronic acid. In an embodiment, the chelant is ethylenediaminedisuccinic acid.

The first and second compositions comprise a viscosity-increasing agent. The viscosity-increasing agent is selected from the group consisting of: clays; polysaccharides; silicas; block copolymers of polypropylene oxide (PPO) and polyethylene oxide (PEO); and mixtures thereof. The first composition and the second composition comprise from about 0.2% to about 20% of viscosity-increasing agent, by total weight of the respective composition. In an embodiment,

the first composition and the second composition comprise from about 0.2% to about 10%, from about 0.3% to about 5%, from about 0.4% to about 3%, or from about 0.60%, or from about 0.70%, or from about 0.80%, or from about 0.90%, or from about 1.0%, or from about 1.1%, or from about 1.2%, or from about 1.3%, or from about 1.4%, or from about 1.5%, or from about 1.6%, or from about 1.7%, or from about 1.8%, or from about 1.9%, or from about 2.0%, or from about 2.1%, or from about 2.2%, or from about 2.3% or from about 2.5% to about 2.9%, or to about 2.8%, or to about 2.7%, or to about 2.6%, or to about 2.5%, or to about 2.4%, or to about 2.3%, or to about 2.2%, or to about 2.1 %, or to about 2.0% of viscosity-increasing agent, by total weight of the respective composition. In an embodiment, these amounts are the total amount of viscosity-increasing agent.

In an embodiment, the viscosity-increasing agent comprises: hydrophobic blocks of polypropylene oxide (PPO) and hydrophilic blocks of polyethylene oxide (PEO). In an embodiment, the viscosity-increasing agent is a hydrosoluble thermosensitive polymer which comprises polyoxyalkylene triblock linear chains in more than 80% by weight of said thermosensitive polymer, said linear chains consisting of blocks of poly(ethylene oxide) (PEO) and of blocks of poly(propylene oxide) (PPO) in the form of PEO-PPO-PEO and conforming to Formula 1:

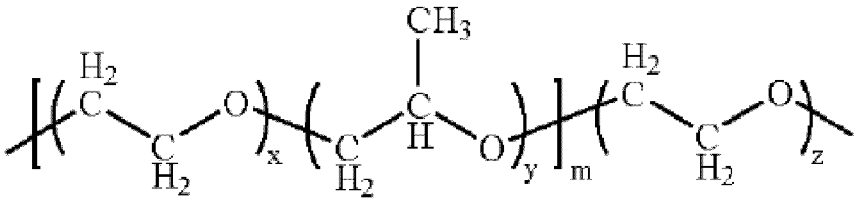

Formula 1

wherein $20<x<120$, $20<y<120$, $20<z<120$, and $m>0$; said linear chains are linked together by one or more organic linking groups which are bonded to said linear chains by either a carbamate bond or a urea bond; and both said carbamate bond and urea bond are present in said polymer. Herein a "hydrosoluble thermosensitive polymer" means the viscosity of the polymer changes reversibly as a function of their temperature. In an embodiment, "hydrosoluble thermosensitive polymer" means a polymer whereby when the polymer is dissolved as a 10% solution (by weight) in water, the viscosity of the polymer (in Pa.s) can increase by at least a factor of 10, or of 100, or of 1000 when the temperature increases above 25°C. In an embodiment, the viscosity-increasing agent is a hydrosoluble thermosensitive polymer conforming to Formula II:

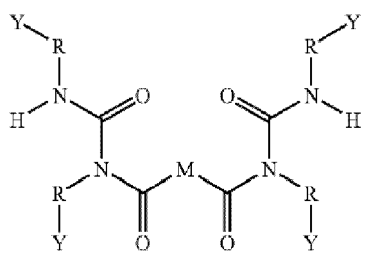

Formula 2

wherein, M is a thermosensitive polymer which comprises polyoxyalkylene triblock linear chains in more than 80% by weight of said thermosensitive polymer, said linear chains consisting of blocks of poly(ethylene oxide) (PEO) and of blocks of poly(propylene oxide) (PPO) in the form of PEO-PPO-PEO conforming to Formula 1 (see above), wherein $20<x<120$, $20<y<120$, $20<z<120$, and $m>0$; said linear chains are linked together by one or more organic linking groups which are bonded to said linear chains by either a carbamate bond or a urea bond; and both said carbamate bond and urea bond are present in said polymer; R is an organic linking group selected from the group consisting of:

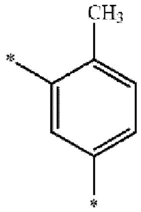 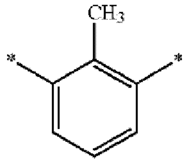 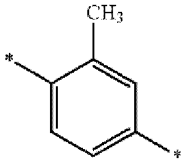

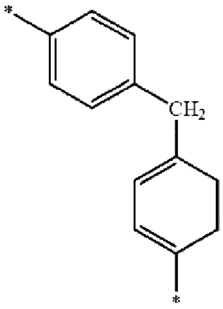 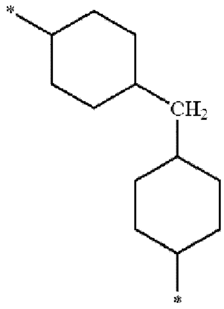 and 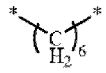 ;

Y is a terminal ethyl or methyl function, an amino group, or another thermosensitive polymer; where R is bonded with Y via a carbamate bond and/or a urea bond. Such polymers are described in US7339013B2, which is incorporated by reference herein.

In an embodiment, the viscosity-increasing agent is a clay. In an embodiment, the clay is selected from the group consisting of: laponite clays, bentonite clays, and mixtures thereof. In an embodiment, the composition/formulation comprirses from about 1% to about 10%, or from about 2% to about 7% clay. IN an embodiment, the viscosity-increasing agent is a clay and the clay is selected from the group consisting of: laponite clays, bentonite clays, and mixtures thereof and the first composition and the second composition respectively comprirse from about 2% to about 7% clay. In an embodiment, the viscosity-increasing agent is a silica.

In an embodiment, the viscosity-increasing agent is a polysaccharide. In an embodiment, the viscosity-increasing agent is a polysaccharide and the polysaccharide is selected from the group consisting of: hydroxyethylcellulose, hydroxypropylcellulose, starch compounds, xanthan gum, carrageenans, and mixtures thereof. In an embodiment, the viscosity-increasing agent is a heteropolysaccharide. The total polysaccharide content present in the formulation/composition may be from about 0.2% to about 5%, or from about 0.5% to about 4%. Suitable polysaccharides and heterosaccharides include starches and derivatives thereof, e.g. mono- or di-esters with phosphoric acid, cellulose types and their derivatives, xanthan gums, carrageenans. Heteropolysaccharides include xanthan gum such as Keltrol® from Kelco, and Natrosol® 250 HHR from Herkules. In an embodiment, the sole viscosity-increasing agent is a xanthan gum or derivative thereof, and wherein the first and/or second composition comprises: from about 0.2% to about 2%, or from about 0.4% to about 1.9%, or from about 0.6% to about 1.5%, of xanthan gum or derivative thereof. In an embodiment, the viscosity-increasing agent is a starch compound or derivatives thereof, and wherein the first and/or second composition comprises from about 0.3% to about 4% total starch content. In an embodiment, the viscosity-increasing agent is a starch compound. In an embodiment, the viscosity-increasing agent is a hydroxypropyl starch phosphate. An example of a hydroxypropyl starch phosphate is Structure® XL from Akzo Nobel.

Suitable viscosity-increasing agents include: Expert Gel® EG 56 (Bis-methoxy PEG-13 PEG-438/PPG-110 SMDI Copolymer) available from PolymerExpert S.A., Pessac, France; Viscarin® TP 389 (carrageenan, silica) available from FMC Corporation, Brussels, Belgium; Keltrol® CGT (Xanthan gum / Polysaccharide) available from CP Kelco, Georgia, USA; Structure® XL (Hydroxypropyl Starch Phosphate) available from Akzo Nobel; Aerosil® 200 (Hydrophilic fumed silica) available from Evonik Industries (Hanau-Wolfgang, Germany); Jaguar® HP (Guar gum / Polysaccharide) available from Rhodia; CMC sodium salt (carboxymethylcellulose); Laponite® XLG (Laponite) available from Rockwood Additives Ltd (Cheshire, UK); Laponite® XLS (Bentonite) available from Rockwood Additives Ltd; Thixogel® MP 100 (Quaternium-18 Bentonite, non irradiated) available from Rockwood Additives Ltd; Bentone® 34 (bentonite) available from Elementis; Thixogel® VP (Quatemium-90 Bentonite, non irradiated) available from Rockwood Additives Ltd.

In an embodiment, the viscosity-increasing agent is pre-incubated in water before incorporation into a composition or formulation as described herein.

In an embodiment, the viscosity-increasing agent is not an associative thickening polymer. In an embodiment, the composition/formulation (e.g. first and/or second compositions) is substantially free of an associative thickening polymer. In an embodiment, the composition/formulation (e.g. first and/or second compositions) is substantially free of: Acrylates/Ceteth-20 Itaconate Copolymers, Polyurethane-39 polymers, Acrylates/Beheneth-25 Methacrylate Copolymers, Acrylates/C10-30 Alkyl Acrylate Crosspolymers, Sodium Acrylate/Sodium Acryloyldimethyl Taurate Copolymers, and mixtures thereof.

A formulation/composition herein, in particular the colour formulation, may have a viscosity of from 1,000 to 60,000 cPs, or from 2,000 to 30,000 cPs, or from 3,000 to 25,000 cPs, measured at at 26.7°C, 1 rpm, with a Brookfield viscometer. Viscosity is measured using Brookfield viscometers with cone and plate attachment. For viscosities in the range of 0-12000 cPs the Brookfield DV-11 viscometer with S42 plate is used. 2ml sample of the composition is equilibrated at 26.7°C for three minutes before the readings are taken at 1 rpm. For viscosities in the range of 12,000-60,000 cPs the Brookfield DV-1 viscometer with S52 plate is used. 0.5ml sample of the composition is equilibrated for 1 minute at 26.7°C before the readings are taken at 1 rpm.

In an embodiment, the composition/formulation comprises a hydrophobic phase. In an embodiment, the hydrophobic phase comprises: fatty alcohols, fatty acids, or mixtures thereof. In an embodiment, the fatty alcohols and/or fatty acids comprise from 10 to 30, or from 12 to 20, or from 16 to 18 carbon atoms. In an embodiment, the first and/or second formulation comprises from about 0.1% to about 80%, or from about 5% to about 50%, or from about 10% to about 30% of fatty components. In an embodiment, the colour formulation comprises a hydrophobic phase. In an embodiment, the hydrophobic phase comprises two different fatty alcohols. In an embodiment, the hydrophobic phase comprises two different fatty alcohols, both comprising from about 10 to about 14 carbons.

In an embodiment, the composition/formulation comprises a hydrophilic phase. The hydrophilic phase may be in the form of a cosmetically acceptable carrier, for example an aqueous cosmetically acceptable carrier. A composition and/or formulation as described herein may comprise a cosmetically acceptable carrier. The composition/formulation may comprise from about 60% to about 99.9%, or from about 70% to about 95%, or from about 80% to about 90%, of a cosmetically acceptable carrier, by total weight of the composition/formulation. The cosmetically acceptable carrier may comprise water; silicones such as volatile silicones, amino or non-amino silicone gums; organic compounds such as $C_2$-$C_{10}$ alkanes, acetone, methyl ethyl ketone, volatile organic $C_1$-$C_{12}$ alcohols, esters of $C_1$-$C_{20}$ acids and of $C_1$-$C_8$ alcohols such as methyl acetate, butyl acetate, ethyl acetate, and isopropyl myristate, dimethoxyethane, diethoxyethane, $C_{10}$-$C_{30}$ fatty alcohols such as lauryl alcohol, cetyl alcohol, stearyl alcohol, and behenyl alcohol; $C_{10}$-$C_{30}$ fatty acids such as lauric acid and stearic acid; $C_{10}$-$C_{30}$ fatty amides such as lauric diethanolamide; $C_{10}$-$C_{30}$ fatty alkyl esters such as $C_{10}$-$C_{30}$ fatty alkyl benzoates; hydroxypropylcellulose, and mixtures thereof. In an embodiment, the carrier comprises water, fatty alcohols, volatile organic alcohols, and mixtures thereof. In an embodiment, the cosmetically acceptable carrier is water.

A composition and/or formulation as described herein may comprise a pH modifier and/or buffering agent in an amount that is sufficiently effective to adjust the pH of the composition/formulation to fall within a range from about 3 to about 13, or from about 7 to about 12, or to about 11, or to about 10, or to about 9, or to about 8. In an embodiment, the first composition and/or second composition has a pH of about 7 to about 9. In some embodiments, the pH range for the carbonate ion source as described herein below is from about 8.5 to about 9.5, or from about 8.0 to about 9.0. Suitable pH modifiers and/or buffering agents for use herein include, but are not limited to: ammonia, alkanolamines such as monoethanolamine, diethanolamine, triethanolamine, monopropanolamine, dipropanolamine, tripropanolamine, tripropanolamine, 2-amino-2-methyl-1-propanol, and 2-amino-2-hydroxymethyl-1,3,-propandiol and guanidium salts, alkali metal and ammonium hydroxides and carbonates, preferably sodium hydroxide, sodium silicate, sodium meta silicate and ammonium carbonate, and acidulents such as inorganic and inorganic acids, e.g., phosphoric acid, acetic acid, ascorbic acid, citric acid or tartaric acid, hydrochloric acid, and mixtures thereof.

A composition and/or formulation as described herein may comprise an alkalising agent. By "alkalising agent" it is meant one or more compound suitable for raising the pH to alkaline level, in particular to a pH between 9 and 11. Generally, the most commonly used alkalising agent in the art is ammonia. Non-ammonia alkalising agents are also known and preferred due to reduced olfactory stimulation. For example, alkanolamines such as monoethanolamine. A composition and/or formulation as described herein may comprise a non-ammonia alkalising agent selected from the group consisting of: monoethanolamine (MEA), sodium silicate, sodium meta silicate, diethanolamine, triethanolamine, monopropanolamine, dipropanolamine, tripropanolamine, 2-amino-2-methyl-1,3-propanediol, 2-amino-2-methyl-1-propanol (a.k.a. aminomethylpropanol, AMP), 2-amino-2-hydroxymethyl-1,3-propanediol, and mixtures thereof. Monoethanolamine (MEA) or aminomethylpropanol (AMP) are commonly used in ammonia-free hair dye products and may be preferred as the alkalising agent alone or in combination with each other or other alkalising agents. Monoethanolamine may be in particular be preferred to be used alone or in combination with other non-ammonia alkalising agent. A composition and/or formulation as described herein may comprise ammonia in addition to the non-ammonia alkalising agent, for example less than 0.5% ammonia. In an embodiment, the alkalising agent is monoethanolamine (MEA). In an embodiment, the first and/or second composition comprises the alkalising agent monoethanolamine (MEA). In an embodiment, the first and/or second composition comprises the alkalising agent monoethanolamine (MEA) and the oxidative dye compound is a mixture of 2-methoxymethyl-1,4-benzenediamine and a coupler.

In an embodiment, the composition/formulation comprises a surfactant. In an embodiment, the first composition and/or the second composition comprises a surfactant. The first composition and/or the second composition may comprise from about 0.001% to about 10%, or from about 0.1% to about 8%, or from about 0.5% to about 5%, or from about 0.4% to about 2%, or from about 0.8% to about 1.5%, of a surfactant. The surfactant may be selected from the group

consisting of: anionic surfactants, amphoteric surfactants, a zwitterionic surfactants, a cationic surfactants, a non-ionic surfactants, or mixtures thereof. The surfactant is useful for stabilising the hydrophobic phase in the composition, for example stabilising the gel network and/or lamellar structure. In an embodiment, the anionic co-surfactant is sodium lauryl sulfate or sodium laureth sulfate. In an embodiment, the surfactant is a non-ionic surfactant. The non-ionic surfactant may be selected from the group consisting of lanolin alcohol, and polyoxyethylene ethers of fatty alcohols, and mixtures thereof. In an embodiment, the non-ionic surfactant is ceteareth-n, wherein n is from about 2 to about 100, or from about 10 to about 30.

In an embodiment, the composition/formulation is substantially free of: a cationic polymer and/or cationic surfactant. In an embodiment, the composition/formulation is substantially free of a silicone compound.

The first composition and/or the second composition may be in the form of a cream or an emulsion. In an embodiment, the first composition and the second composition respectively have a lamellar structure; wherein the lamellar structure is sandwich of a hydrophobic phase and a hydrophilic phase; and wherein the hydrophilic phase comprises the viscosity-increasing agent; wherein the wherein the lamellar structure has a d-spacing; wherein the d-spacing is the total thickness of a hydrophobic layer and a hydrophilic layer; wherein the d-spacing is from about 12 nm to about 40 nm. The existence of lamellar structure can be observed by cryo-scanning electronic microscopy (cryo-SEM). The lamellar structure can be measured by analyzing an SEM picture, for example, by calculating area of lamellar structure per unit area. D-spacing can be defined according to the following equation:

$$\text{d-spacing} = d_{water} + d_{bilayer}$$

where $d_{water}$ is the hydrophilic phase and $d_{bilayer}$ is the hydrophobic phase. D-spacing can be measured by using a High Flux Small Angle X-ray Scattering Instrument available from PANalytical with a trade name SAXSess, under the typical conditions of Small Angle X-Ray Scattering (SAXS) measurements in a q-range ($q=4\pi/\lambda\sin(\theta)$ wherein $\lambda$ is the wavelength and $\theta$ is half the scattering angle) of $0.06<q/nm^{-1}<27$ which corresponds to $0.085<2\theta/degree<40$. The data are transmission calibrated by monitoring the attenuated primary beam intensity and normalizing it into unity, so that relative intensity for different samples can be obtained. The transmission calibration allows us to make an accurate subtraction of water contribution from the net sample scattering. D-spacing is calculated according to the following equation (which is known as Bragg's equation):

$$n\lambda=2d\sin(\theta),$$

wherein n is the number of lamellar bilayers (i.e. hydrophobic phases)

In an embodiment, the d-spacing is from about 15 nm to about 35 nm, or from about 20 nm to about 30 nm, or from about 25 nm to about 28 nm.

In an embodiment, the first and second compositions are substantially free of anionic surfactants and anionic polymers. It is useful for the compositions to be free of such anionics in view of the stability of the lamellar structure.

In an embodiment, the "coated hair fibre portions" are those of doll heads or mannequin heads. In an embodiment, the "coated hair fibre portions" are those comprising synthetic hair. In an embodiment, the "coated hair fibre portions" comprise keratin fibres.

In an embodiment, the first and/or second composition is obtained from mixing together a colour formulation and a developing formulation. In an embodiment, the first and/or second composition is obtained from mixing together a colour formulation and a developing formulation, wherein the colour formulation comprises a hair colouring agent, and wherein the hair colouring agent is a mixture of at least one primary intermediate and at least one coupler, and wherein the developing formulation comprises: from about 0.5% to about 6% viscosity-increasing agent and wherein the viscosity-increasing agent is selected from the group consisting of: clays; polysaccharides; silicas; block copolymers of polypropylene oxide (PPO) and polyethylene oxide (PEO); and mixtures thereof; from about 1% to about 15% of an oxidising agent; optionally from about 1% to about 10% of a fatty alcohol; optionally from about 0.1% to about 2% of a surfactant; and QSP water. In an embodiment, the weight ratio of the colour formulation to the developing formulation (i.e. colour formulation:developing formulation) is from about 1:5 to about 5:1. In an embodiment, the colour formulation comprises a hair colouring agent, and wherein the hair colouring agent is a mixture of at least one primary intermediate and at least one coupler, and wherein the developing formulation comprises hydrogen peroxide. In an embodiment, the thickening formulation comprises a viscosity-increasing agent and water. In an embodiment, the weight ratio of the colour formulation to the developing formulation to the thickening formulation (i.e. colour formulation:developing formulation:thickening formulation) is from about 10:10:0.5 to about 10:10:2, or from about 10:20:0.5 to about 10:20:2.

The method relates to the formation of a first plurality of coated hair fibre portions. As used herein, a "hair fibre portion" may be an entire fibre of hair from root to tip, alternatively it may relate only to a section of this fibre e.g. only the root section, or only the tip section. A "plurality of hair fibre portions" as used herein, relates to two or more hair fibre portions. Typically a plurality of hair fibre portions relates to a bundle of hair fibre portions, which have been gathered together such they are in close proximity i.e. in a bundle. The respective roots from which the bundle originates may also be in close proximity. For longer hair, however, this may not be the case. A plurality of hair fibre portions may relate to, for example, a bundle of circa 10 entire hair fibres from root to tip. Alternatively, a plurality of hair fibre portions may relate to a bundle of root portions of circa 50 hair fibres. The alternatives in the above description are due to variations in the colouring effects needed in order to create a specific hairstyle. For example, the target final hair effect may require a first hair colouring agent to be applied to circa 50% of the root portions, a second hair colouring agent to be applied to the remaining 50% of the root portions, and a third colouring agent to be applied to all of the tip portions. The term "coated hair fibre portion" as used herein means a section of a hair fibre that has been covered or coated, preferably over the majority of its surface area, with a composition. In an embodiment, the phrase "coated hair fibre portions, wherein the coating comprises a composition," means "hair fibre portions coated with a composition". The method comprises the formation of a first plurality of coated hair fibre portions, and subsequently styling the hair, wherein the first plurality of coated hair fibre portions is contacted with a second plurality of coated hair fibre portions. "Contacted with" , as used herein, means placing two elements in close proximity such that they touch each other.

In an embodiment, second hair colouring agent is different from the first hair colouring agent. In an embodiment, the first hair colouring agent exhibits a different final colour result versus the final colour result exhibited by the second hair colouring agent. In an embodiment, the first plurality of coated hair fibre portions and the second plurality of coated hair fibre portions do not originate from substantially the same plurality of hair fibre roots. In an alternative embodiment, the first plurality of coated hair fibre portions and the second plurality of coated hair fibre portions originate from substantially the same plurality of hair fibre roots. For example, the same plurality of hair fibre portions may be coated with both the first composition and the second composition. This may mean that the same plurality of hair fibre portions is dyed with different colours. For example the first plurality of hair fibre portions could be the root portion of the plurality of hair fibres, and this root portion could be coated with the first composition and the second plurality of hair fibre portions could be the remaining portion of the plurality of hair fibres including the tip portion, and this remaining portion could be coated with the second composition.

In an embodiment, a first, a second, a third and a fourth plurality of coated hair fibre portions are formed, wherein the coating comprises a composition as per the second aspect. In an embodiment, the composition for each coating comprises two or more different hair colouring agents, wherein the hair colouring agents may result in a different final colour result.

In an embodiment, the plurality of coated hair fibre portions are laid flat onto the head. In an embodiment, the plurality of coated hair fibre portions are in slice-shaped form. In an embodiment, the first plurality of coated hair fibre portions is sculpted to form a curl.

"Substantially the same", as used herein, means at least 50% the same, or more than 60% the same, or more than 70% the same, or more than 80% the same, or more than 90% the same, or more than 95% the same.

In an embodiment, the method results in a hairstyle and wherein the hairstyle is selected from the group consisting of: turban; plait; braid; tousle; wave; criss-cross; blending; 2-step; sculpting; and combinations thereof.

In an embodiment, prior to the formation of the second plurality of coated hair fibre portions, first plurality of hair fibre portions is styled to form a hairstyle. In an embodiment, subsequent to the formation of the second plurality of coated hair fibre portions, the second plurality of coated hair fibre portions is styled to form a hairstyle.

In an embodiment, the first hair colouring agent and second hair colouring agent are allowed to remain on the hair for a development time. The development time may be from about 1 min to about 90 min, or from about 5 min to about 70 min, or from about 10 min to about 60 min, or from about 10 min to about 40 min. In an embodiment of the method, the compositions are rinsed from the hair after the development time has expired. In an embodiment, the hair is treated with a treatment during the development time. The treatment may selected from the group consisting of the exposure of the hair: to a temperature of 20°C to 45°C, or 30°C to 42°C for at least 0.5 min, or at least 1 min, or at least 3 min, or at least 5 min; to a relative humidity of 20% to 80%, or 40% to 70% for at least 0.5 min, or at least 1 min, or at least 3 min, or at least 5 min; and combinations thereof.

Following hair rinsing, the hair displays a hair colour effect. The hair colour effect may be selected from the group consisting of: hair strand effects, highlighting, lowlighting, and combinations thereof.

The method does not comprise the application of a solid barrier means in order to separate the first plurality of coated hair fibre portions from the second plurality of coated hair fibre portions. "Solid barrier means", as used herein, means that a solid substance is placed on the hair, such that portions of the hair are not able to touch each other and thus no staining is possible. Foils are an example of solid barrier means, which are typically used such that portions of hair are individually wrapped in foil. Another example of a solid barrier means is a cap comprising holes. Such a cap is described, in particular in Fig.2 and in §0004 on p.2, in European patent application EP1969961A2, filed on 10th March

2008 in the name of the Procter & Gamble Co. and published on 17th Sept 2008. In an embodiment, the solid barrier means is a physical barrier selected from the group consisting of foil, plastic, film, cotton wool, padding material, caps, and combinations thereof. In an embodiment, the method comprises the application of a liquid barrier means in order to separate the first plurality of coated hair fibre portions from the second plurality of coated hair fibre portions and such that no staining occurs. The liquid barrier means may be a conditioning formulation. In another embodiment, method does not comprise the application of any barrier means in order to separate the first plurality of coated hair fibre portions from the second plurality of hair fibre portions. "Any barrier means" comprises liquid barrier means and solid barrier means.

According to the second aspect, the present invention relates to hair colouring composition being in ready to use form; wherein the composition comprises a hair colouring agent, an oxidising agent, a viscosity-increasing agent, water and optionally a fatty alcohol; wherein the hair colouring agent is a mixture of at least one primary intermediate and at least one coupler; and wherein the viscosity-increasing agent is selected from the group consisting of: clays; polysaccharides; silicas; block copolymers of polypropylene oxide (PPO) and polyethylene oxide (PEO); and mixtures thereof; and wherein the composition comprises from about 0.2% to about 20% of viscosity-increasing agent. In an embodiment, the hair colouring composition has a lamellar structure; wherein the lamellar structure is sandwich of hydrophobic layers and hydrophilic layers; wherein the wherein the lamellar structure has a d-spacing; wherein the d-spacing is the total thickness of a hydrophobic layer and a hydrophilic layer; wherein the d-spacing is from about 12 nm to about 40 nm; wherein the hydrophobic layer comprises a fatty alcohol; wherein the hydrophilic layer comprises water and wherein the hydrophilic layer comprises the viscosity-increasing agent. Lamellar structures, d-spacing and how they are measured are described hereinabove. In an embodiment, the hydrophilic layer comprises least 70% water, by total weight of the hydrophilic layer. The details disclosed herein in relation to the compositions/formulations of the other aspects, are also applicable to the second aspect.

According to the third aspect, the present invention relates to the use of the composition according to the second aspect, for colouring and/or styling hair. The details disclosed herein in relation to the compositions/formulations of the other aspects, are also applicable to the third aspect.

According to the fourth aspect, the present invention relates to a developing formulation comprising:

- from about 0.5% to about 10% viscosity-increasing agent and wherein the viscosity-increasing agent is selected from the group consisting of: clays; polysaccharides; silicas; block copolymers of polypropylene oxide (PPO) and polyethylene oxide (PEO); and mixtures thereof;
- from about 1% to about 15% of an oxidising agent;
- optionally from about 1% to about 10% of a fatty alcohol;
- optionally from about 0.1% to about 2% of a surfactant;
- water.

In an embodiment, the oxidising agent is hydrogen peroxide. The details disclosed herein in relation to the compositions/formulations of the other aspects, are also applicable to the fourth aspect.

According to the fifth aspect, the present invention relates to a process for creating a hair colouring composition comprising mixing: (a) a developing formulation according to the fourth aspect; (b) a hair colour formulation comprising a hair colouring agent wherein the hair colouring agent is a mixture of at least one primary intermediate and at least one coupler. In an embodiment, the colour formulation is substantially free of a viscosity-increasing agent, wherein the viscosity-increasing agent is selected from the group consisting of: clays; polysaccharides; silicas; block copolymers of polypropylene oxide (PPO) and polyethylene oxide (PEO); and mixtures thereof. In an embodiment, the colour formulation further comprises a direct dye. The details disclosed herein in relation to the compositions/formulations of the other aspects, are also applicable to the fifth aspect.

According to the sixth aspect, the invention relates to a kit comprising: (a) application instructions comprising the method according to the first aspect; (b) a composition. In an embodiment, the composition is selected from the group consisting of: the hair colouring composition as described herein (see second aspect); the developing formulation as described herein (see fourth aspect). The details disclosed herein in relation to the compositions/formulations of the other aspects, are also applicable to the sixth aspect.

According to a seventh aspect, the invention relates to a colour formulation comprising:

(a) from about 0.5% to about 10% viscosity-increasing agent and wherein the viscosity-increasing agent is selected from the group consisting of: clays; polysaccharides; silicas; block copolymers of polypropylene oxide (PPO) and polyethylene oxide (PEO); and mixtures thereof;
(b) a hair colouring agent wherein the hair colouring agent is a mixture of at least one primary intermediate and at least one coupler; (c) optionally an alkalising agent; (d) optionally from about 1% to about 10% of a fatty alcohol (e) water; and wherein the colour formulation is substantially free of peroxide and persulfate. In an embodiment, the colour formulation is also substantially free of oxidising agent. The details disclosed herein in relation to the compositions/formulations of the other aspects, are also applicable to the seventh aspect.

EXAMPLES

The following examples are colour formulations, developing formulations and viscosity-increasing agents for obtaining first and/or second compositions as described herein.

Colour formulations A to P

| Colour Formulation | Colour result | Hair colouring agent | Dye and salt load | Alkalising agent § | Other components§ |
|---|---|---|---|---|---|
| A | black | Oxidative dye compounds | highest dye load, high electrolyte | low ammonia and MEA | Hydrophobic phase comprising hydrophobic components (20% to 28%); hydrophilic phase comprising water (50% to 76%); surfactant (4% to 6%); and alkalising agent(s) (3% to 11 %). |
| B | neutral dark brown | Oxidative dye compounds | medium high dye load | medium low ammonia | |
| C | eutral light blond | Oxidative dye compounds | low dye load | high ammonia | |
| D | light brown e.g. hazelnut | Oxidative dye compounds | medium dye load | medium ammonia | |
| E | intense red shade | Oxidative dye compounds | high concentration of the dye pyrazole | high ammonia | |
| F | intense red shade | Oxidative dye compounds | low concentration of the dye pyrazole | high ammonia | |
| G | gold blond | Mixture of oxidative dye compounds and direct dyes | low dye load | high ammonia | |
| H | intense red shade | Direct dyes | high dye load | - | Hydrophilic phase, surfactant |
| I | gold shade | Direct dyes | low dye load | - | |
| J | Any of colour formulations A to G | Oxidative dye compounds | As per selected colour formulations | 3% to 11% ammonia | Hydrophobic phase comprising hydrophobic components (8 to 10%); hydrophilic |
| | | | A to G | | phase comprising water (75 to 90%); surfactant (2% to 3%). |
| K | Any of colour formulations A to G | Oxidative dye compounds | As per selected colour formulations A to G | 3% to 11% ammonia | Hydrophobic phase comprising hydrophobic components (2 to 10%); hydrophilic phase comprising water (75 to 95%); surfactant (1% to 3%). |

(continued)

| | | | | | |
|---|---|---|---|---|---|
| L | Any of colour formulations A to G | Oxidative dye compounds | As per selected colour formulations A to G | 4 to 8% MEA | Hydrophobic phase comprising hydrophobic components (8 to 12%); hydrophilic phase comprising water (70 to 85%); surfactant (6% to 8%). |
| M | Optionally any of colour formulations A to G | Persulfate and optionally oxidative dye compounds | Optionally as per selected colour formulations A to G. | Up to 2 % ammonia | Powder. |
| N | Any of colour formulations A to G | Oxidative dye compounds | As per selected colour formulations A to G | 3% to 11% ammonia | Hydrophobic phase comprising hydrophobic components (30 to 45%); hydrophilic phase comprising water (40 to 64%); surfactant (6% to 15%). |
| O | Any of colour formulations H to I | Oxidative dye compounds | As per selected colour formulations H to I | - | Hydrophobic phase comprising hydrophobic components (30 to 45%); hydrophilic phase comprising water (40 to 65%); surfactant (6% to 15%). |
| P | Any of colour formulations G | Mixture of oxidative dye compounds and direct dyes | As per colour formulation G | MEA | Hydrophobic phase comprising hydrophobic components (30 to 45%); hydrophilic phase comprising water (40 to 65%); surfactant (6% to 15%). |
| KEY: § = stated numbers are percent calculated by w/w of the colour formulation. | | | | | |

Developing Formulations I to V

| Developing Formulation | Concentration of oxidising agent§ | Concentration of viscosity-increasing agent§ | Other components § |
|---|---|---|---|
| I | 1% | - | Hydrophobic phase comprising cetearyl alcohol (2% to 6 %); surfactant (0.6% to 0.8%) ; hydrophilic phase comprising water (93% to 97%) |
| II | 4% | - | |
| IIIa | 6% | - | |
| IIIb | 6% | 1% | |
| IIIc | 6% | 2% | |
| IV | 9% | - | |
| Va | 12% | - | |
| Vb | 12% | 1% | |
| Vb | 12% | 2% | |
| KEY: § = stated numbers are percent calculated by w/w of the developing formulation. | | | |

Viscosity-increasing agents

#1) Expert Gel® EG 56 (Bis-methoxy PEG-13 PEG-438/PPG-110 SMDI Copolymer);
#2) Viscarin® TP 389 (Carrageenan) ;
#3) Keltrol® CGT (Xanthan gum);
#4) Structure® XL (Hydroxypropyl Starch Phosphate);
#5) Aerosil® 200 (Silica);
#6) Jaguar® HP (Guar gum / Polysaccharide);
#7) CMC sodium salt (Carboxymethylcellulose);
#8) Laponite® XLG (Laponite);
#9) Laponite® XLS (Bentonite);
#10) Thixogel® MP 100 (Quaternium-18 Bentonite, non irradiated);
#11) Bentone® 34 (Bentonite);
#12) Thixogel® VP (Quaternium-90 Bentonite, non irradiated).

DATA

Experiment 1 - Long-lasting hold evaluation

The long lasting hold measurements are observations of the height changes of hair tresses over time at elevated humidity (20°C and a humidity of 65% relative humidity). These methods are performed in order to determine the form stability i.e. the amount of hold, of hair tresses treated with the compositions pursuant to the present invention. With a height calliper comprising a laser, the height changes of tresses are detected, which indicate the amount of hold provided by the compositions pursuant to the present invention. A colour formulation, a developing formulation and, when used, a viscosity-increasing agent (see above Examples section) are mixed to form the final composition. First, the viscosity-increasing agent is mixed with the developing formulation in the indicated amount. The developing formulation and the colour formulation are then mixed 1:1 to form the final composition. A control composition comprises no viscosity-increasing agent in the developing composition and thus is not pursuant to the present invention. The following method is used:

Treatment with each composition: A total of at least 20 g of each composition is thoroughly mixed using a colour brush or in an applicator flask. 5 dry tresses with a length of 170 mm and a dry weight of 2.00 g to 2.02 g are put on a Plexiglas plate. The composition is applied to the tresses. The composition is dispersed equally on the hair tress using a brush such that the hairs of the tress are kept brushed straight and parallel. The final weight of each tress is 6.20 g to 6.23 g.

Measurement procedure: The tresses each have a rubber gatherer at one end, and these rubber ends of the treated tresses are mounted horizontally on a rack. 55 mm in front of the rack is a narrow bar which is parallel and at the same height above the bench as the rack. Each tress lies horizontally from the rack to narrow bar. After the narrow bar a protruding length of 102 mm for each tress is left over. A wet tress (comprising 50% humidity, which would reflect towel dry hair) hangs down loosely from the narrow bar. The composition applied to the tresses provide stiffness (i.e. hold) to the protruding length of the tresses such that they do not hang down loosely from the narrow bar. The extent to which the protruding length of the tress is held is measured by measuring the height of the tip of the tress. The height projection of the tip end of the tress is determined by means of an altimeter with semiconductor laser immediate. The measurement is taken after 0 min, 2 min, 5 min, 10 min, 15 min and 30 min. Taking the length of the tress into account the resulting hold is calculated as follows:

$$\text{hold } [\%] = 100 - ((1_t/102) \text{ x } 100)$$

where $1_t$ = height of horizontally projected tress and
102 mm = protruding length of the tress.

A tress that remained horizontal would have an $1_t$ of 0 mm, and therefore a percentage hold of 100% i.e. this would be the best possible result. A wet tress that hangs down loosely would have an $1_t$ of 102 mm, and therefore a percentage hold of 0% i.e. this would be the worst possible result.

The results of experiment 1 are shown in Figs 1 and 2. Each hold value (indicated as a percentage - see axis Y) is a mean of 5 repeats, since for every composition 5 tresses are utilised. Each Fig indicates the colour formulation, developing formulation and viscosity-increasing agent (see above Examples section) mixed to form the final composition.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is

intended to mean "about 40 mm."

## Claims

1. A method for colouring hair, wherein the method comprises:

    (i) the formation of a first plurality of coated hair fibre portions, by applying a first composition to a first plurality of hair fibre portions;
    wherein the first composition comprises a first hair colouring agent;
    and subsequently
    (ii) styling the hair wherein the first plurality of coated hair fibre portions is contacted with a second plurality of coated hair fibre portions;
    wherein the second plurality of coated hair fibre portions are coated with a second composition;
    wherein the second composition comprises a second hair colouring agent;
    wherein the method does not comprise the application of a solid barrier means in order to separate the first plurality of coated hair fibre portions from the second plurality of coated hair fibre portions;
    wherein the first composition and the second composition are substantially free of persulfate;
    and wherein the first composition and the second composition comprise from about 0.2% to about 20% of viscosity-increasing agent, by total weight of the respective composition; and wherein the viscosity-increasing agent is selected from the group consisting of: clays; polysaccharides; silicas; block copolymers of polypropylene oxide (PPO) and polyethylene oxide (PEO); and mixtures thereof.

2. The method according to claim 1, wherein the second hair colouring agent is different from the first hair colouring agent.

3. The method according to any preceding claim, wherein the viscosity-increasing agent is a hydrosoluble thermosensitive polymer which comprises polyoxyalkylene triblock linear chains in more than 80% by weight of said thermosensitive polymer, said linear chains consisting of blocks of poly(ethylene oxide) (PEO) and of blocks of poly(propylene oxide) (PPO) in the form of PEO-PPO-PEO and conforming to Formula 1:

    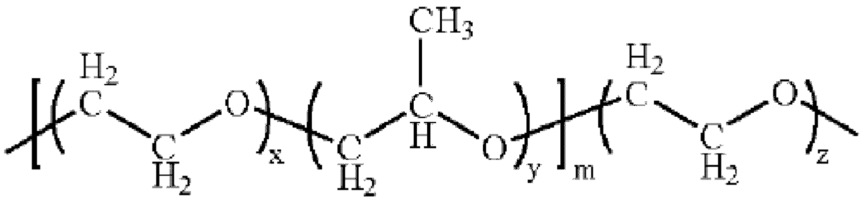

    Formula 1

    wherein $20<x<120$, $20<y<120$, $20<z<120$, and $m>0$; said linear chains are linked together by one or more organic linking groups which are bonded to said linear chains by either a carbamate bond or a urea bond; and both said carbamate bond and urea bond are present in said polymer.

4. The method according to any preceding claim, wherein the first composition and the second composition comprise from about 0.4% to about 3% of viscosity-increasing agent, by total weight of the respective composition.

5. The method according to any preceding claim, wherein the first and/or second hair colouring agents are oxidative dye compounds; and wherein the first and/or second composition comprises an oxidising agent.

6. The method according to any preceding claim, wherein the first composition and the second composition respectively have a lamellar structure; wherein the lamellar structure is sandwich of a hydrophobic phase and a hydrophilic phase; and wherein the hydrophilic phase comprises the viscosity-increasing agent; wherein the wherein the lamellar structure has a d-spacing; wherein the d-spacing is the total thickness of a hydrophobic layer and a hydrophilic layer; wherein the d-spacing is from about 12 nm to about 40 nm.

7. The method according to any preceding claim, wherein the viscosity-increasing agent is a clay and the clay is selected from the group consisting of: laponite clays, bentonite clays, and mixtures thereof and the first composition and the second composition respectively comprirse from about 2% to about 7% clay.

8. The method according to any preceding claim, wherein the viscosity-increasing agent is a polysaccharide and the polysaccharide is selected from the group consisting of:

hydroxyethylcellulose, hydroxypropylcellulose, starch compounds, xanthan gum, carrageenans, and mixtures thereof.

9. The method according to any preceding claim, wherein the first hair colouring agent and/or second hair colouring agent is a mixture of at least one primary intermediate and at least one coupler, wherein the primary intermediate is 2-methoxymethyl-1,4-diaminobenzene and wherein the coupler is selected from the group consisting of: 5-amino-4-chloro-o-cresol, 2,6-diaminopyridine, 2,6-dihydroxyethylaminotoluene, and mixtures thereof.

10. The method according to any preceding claim, wherein the first and/or second composition comprises the alkalising agent monoethanolamine (MEA).

11. A hair colouring composition being in ready to use form;
wherein the composition comprises a hair colouring agent, an oxidising agent, a viscosity-increasing agent, water, and optionally a fatty alcohol; wherein the hair colouring agent is a mixture of at least one primary intermediate and at least one coupler;
and wherein the viscosity-increasing agent is selected from the group consisting of: clays; polysaccharides; silicas; block copolymers of polypropylene oxide (PPO) and polyethylene oxide (PEO); and mixtures thereof;
and wherein the composition comprises from about 0.2% to about 20% of viscosity-increasing agent.

12. The composition according to claim 11, wherein the composition comprises from about 1.0% to about 2.0% of viscosity-increasing agent.

13. Use of the composition according to any of claims 11 to 12, for colouring and/or styling hair.

14. A developing formulation comprising:

- from about 0.5% to about 10% viscosity-increasing agent and wherein the viscosity-increasing agent is selected from the group consisting of: clays; polysaccharides; silicas; block copolymers of polypropylene oxide (PPO) and polyethylene oxide (PEO); and mixtures thereof;
- from about 1% to about 15% of an oxidising agent;
- optionally from about 1% to about 10% of a fatty alcohol;
- optionally from about 0.1% to about 2% of a surfactant;
- water.

15. A process for creating a hair colouring composition comprising mixing:

a) a developing formulation according to claim 14;
b) a hair colour formulation comprising a hair colouring agent wherein the hair colouring agent is a mixture of at least one primary intermediate and at least one coupler.

FIG. 1

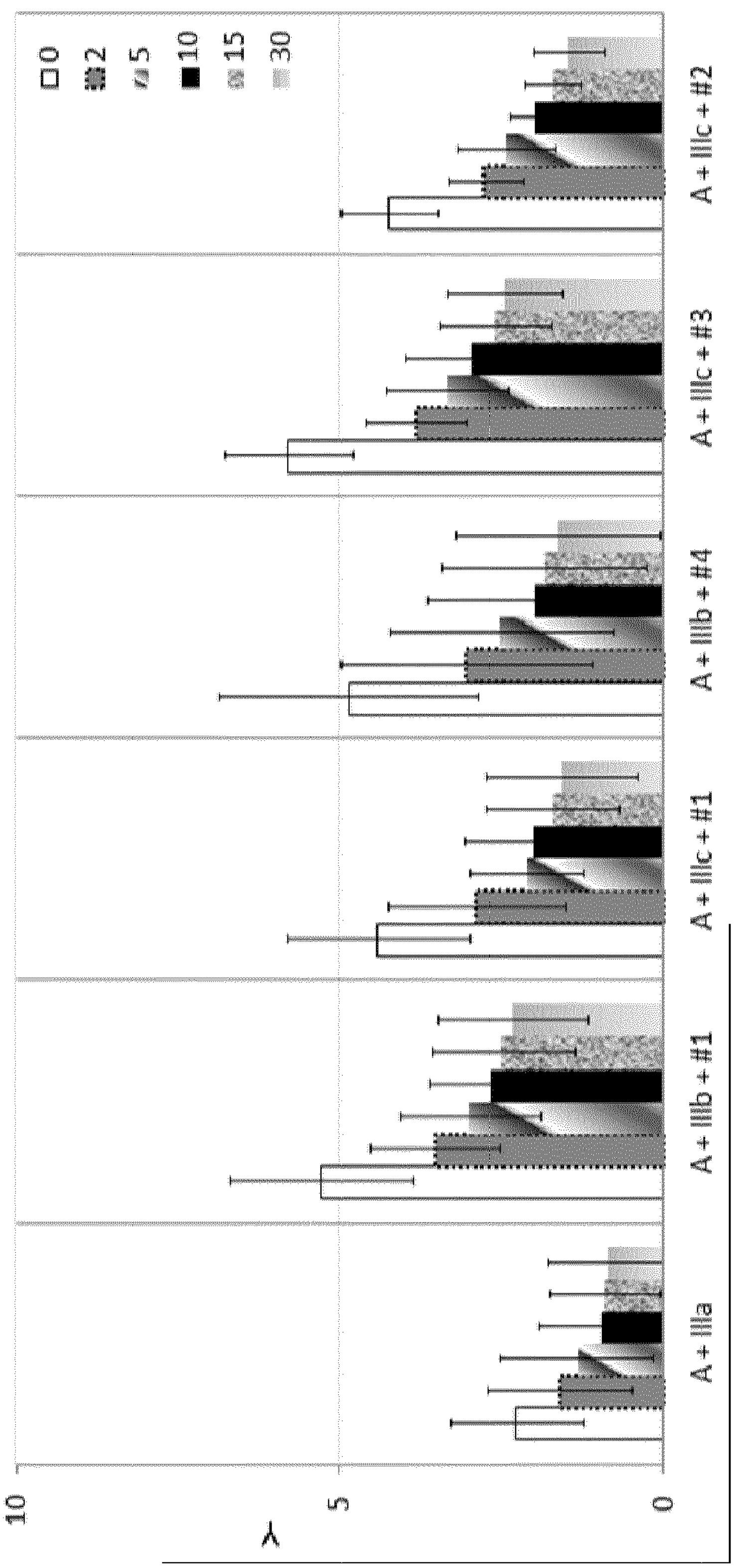

FIG. 2

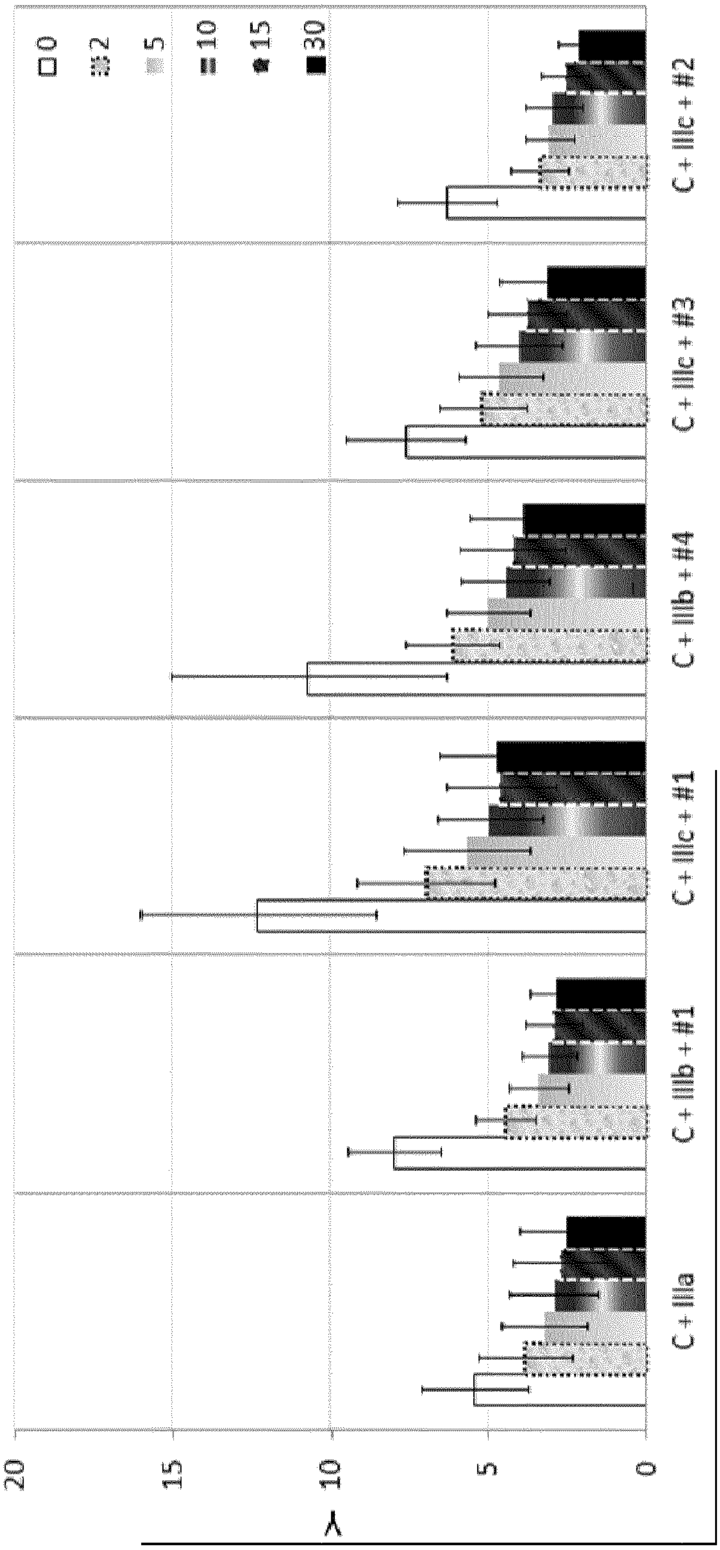
Y
0
5
10
15
20
C + IIIa
C + IIIb + #1
C + IIIc + #1
C + IIIb + #4
C + IIIc + #3
C + IIIc + #2
0
2
5
10
15
30

Europäisches Patentamt
European Patent Office
Office européen des brevets

# EUROPEAN SEARCH REPORT

Application Number
EP 13 16 2464

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2 343 238 A (L'OREAL)<br>7 March 1944 (1944-03-07)<br>* column 3, line 6 - line 45 *<br>* column 16, line 27 - column 18, line 59 *<br>* column 18, line 60 - column 19, line 60 *<br>* examples 1,4 * | 11,13<br>1-15 | INV.<br>A61K8/25<br>A61K8/26<br>A61Q5/10<br>A61K8/73<br>A61K8/86 |
| Y | EP 2 198 846 A1 (L'OREAL)<br>23 June 2010 (2010-06-23)<br>* paragraph [0001] - paragraph [0011] *<br>* paragraph [0017] *<br>* paragraph [0023] *<br>* paragraph [0067] - paragraph [0101] *<br>* claims * | 1-15 | |
| Y | US 2012/317734 A1 (ISP INVESTMENTS)<br>20 December 2012 (2012-12-20)<br>* paragraph [0045] - paragraph [0046] *<br>* paragraph [0066] - paragraph [0072] *<br>* paragraph [0074] - paragraph [0099] *<br>* paragraph [0153] *<br>* examples * | 1-15 | TECHNICAL FIELDS SEARCHED (IPC)<br>A61K<br>A61Q |
| X<br>Y | EP 2 065 073 A2 (L'OREAL)<br>3 June 2009 (2009-06-03)<br>* paragraph [0001] - paragraph [0004] *<br>* paragraph [0011] - paragraph [0012] *<br>* paragraph [0018] - paragraph [0031] *<br>* claims * | 11-15<br>1-15 | |
| A | FR 2 840 907 A1 (POLYMEREXPERT)<br>19 December 2003 (2003-12-19)<br>* page 3, line 4 - page 4, line 11 *<br>* claims * | 1-15 | |

-/--

2

The present search report has been drawn up for all claims

| Place of search | Date of completion of the search | Examiner |
|---|---|---|
| Munich | 28 May 2014 | Irwin, Lucy |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 16 2464

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2011/040632 A2 (KAO CORPORATION) 7 April 2011 (2011-04-07) * paragraph [0001] - paragraph [0005] * * paragraph [0007] - paragraph [0009] * * paragraph [0013] * * examples 13,14 * * claims * ----- | 1-15 | |
| A | MINTEL: "Heads Up! Couture Highlighting Kit", GNPD,, 1 September 2009 (2009-09-01), XP002671827, * Product description * ----- | 1-15 | |
| | | | TECHNICAL FIELDS SEARCHED (IPC) |

2

The present search report has been drawn up for all claims

| Place of search | Date of completion of the search | Examiner |
|---|---|---|
| Munich | 28 May 2014 | Irwin, Lucy |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 16 2464

This annex lists the patent family members relating to the patent documents cited in the above-mentioned European search report. The members are as contained in the European Patent Office EDP file on
The European Patent Office is in no way liable for these particulars which are merely given for the purpose of information.

28-05-2014

| Patent document cited in search report | Publication date | Patent family member(s) | Publication date |
|---|---|---|---|
| US 2343238 A | 07-03-1944 | NONE | |
| EP 2198846 A1 | 23-06-2010 | BR PI0906040 A2 | 29-03-2011 |
| | | CN 101843561 A | 29-09-2010 |
| | | EP 2198846 A1 | 23-06-2010 |
| | | FR 2940102 A1 | 25-06-2010 |
| | | JP 2010143910 A | 01-07-2010 |
| | | US 2010154140 A1 | 24-06-2010 |
| US 2012317734 A1 | 20-12-2012 | NONE | |
| EP 2065073 A2 | 03-06-2009 | BR PI0804841 A2 | 28-07-2009 |
| | | EP 2065073 A2 | 03-06-2009 |
| | | FR 2923388 A1 | 15-05-2009 |
| | | US 2009180979 A1 | 16-07-2009 |
| FR 2840907 A1 | 19-12-2003 | AT 549366 T | 15-03-2012 |
| | | AU 2003258799 A1 | 31-12-2003 |
| | | CA 2489828 A1 | 24-12-2003 |
| | | EP 1521795 A2 | 13-04-2005 |
| | | ES 2384101 T3 | 29-06-2012 |
| | | FR 2840907 A1 | 19-12-2003 |
| | | JP 5237523 B2 | 17-07-2013 |
| | | JP 2005534732 A | 17-11-2005 |
| | | US 2005175573 A1 | 11-08-2005 |
| | | WO 03106536 A2 | 24-12-2003 |
| WO 2011040632 A2 | 07-04-2011 | CN 102548527 A | 04-07-2012 |
| | | EP 2482789 A2 | 08-08-2012 |
| | | JP 2011093883 A | 12-05-2011 |
| | | KR 20120099002 A | 06-09-2012 |
| | | TW 201117833 A | 01-06-2011 |
| | | US 2012128615 A1 | 24-05-2012 |
| | | WO 2011040632 A2 | 07-04-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7339013 B2
- EP 1969961 A2


### Non-patent literature cited in the description


- **SAGARIN.** Cosmetic Science and Technology. Interscience, vol. 2, 308-310